(19) Europäisches Patentamt

European Patent Office

Office européen des brevets

(11) **EP 4 374 783 A1**

(12) # DEMANDE DE BREVET EUROPEEN

(43) Date de publication:
**29.05.2024  Bulletin 2024/22**

(21) Numéro de dépôt: 23207096.1

(22) Date de dépôt: **31.10.2023**

(51) Classification Internationale des Brevets (IPC):
**A61B 5/08** (2006.01)        **A61M 16/00** (2006.01)

(52) Classification Coopérative des Brevets (CPC):
**A61B 5/4818; A61B 5/0826; A61B 5/087;
A61B 5/4833; A61B 5/7267; A61M 16/0051;
A61M 16/026; G16H 20/40; G16H 40/67;
G16H 80/00;** A61M 16/06; A61M 2016/0027;
A61M 2016/0039; A61M 2205/15; A61M 2205/18;

(Cont.)

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**KH MA MD TN**

(30) Priorité:  **25.11.2022   FR 2212318**

(71) Demandeur: **L'AIR LIQUIDE, SOCIETE ANONYME
POUR L'ETUDE ET
L'EXPLOITATION DES PROCEDES GEORGES
CLAUDE
75007 Paris (FR)**

(72) Inventeurs:
• **AMADOU-BOUBACAR, Habiboulaye
78350 Les Loges en Josas (FR)**

• **RAHIM, Mehdi
78350 Les Loges en Josas (FR)**
• **BOURGEOIS, Thomas
78350 Les Loges en Josas (FR)**
• **GUILLEMOT, Mathilde
78350 Les Loges en Josas (FR)**

(74) Mandataire: **Air Liquide
L'Air Liquide S.A.
Direction de la Propriété Intellectuelle
75, Quai d'Orsay
75321 Paris Cedex 07 (FR)**

Remarques:
Revendications modifiées conformément à la règle
137(2) CBE.

(54) **SYSTÈME POUR PRÉDIRE UN RISQUE DE NON-ADHÉRENCE À UN TRAITEMENT DE L'APNÉE DU SOMMEIL**

(57)     L'invention concerne un système (1) pour détecter et alerter d'un risque de non-adhérence à un traitement de l'apnée du sommeil d'un patient (P) souffrant d'apnée du sommeil. Il comprend un dispositif de mesure (2) pour opérer des mesures de débit et/ou de pression d'un gaz respiratoire fourni au patient; des moyens informatiques de traitement de données (4) pour traiter les mesures obtenues sur au moins 3 jours afin de déterminer ou estimer des grandeurs ou valeurs de plusieurs paramètres respiratoires ($p_1$, $p_2$..., $p_i$) du patient considéré; déduire de ces paramètres respiratoires ($p_1$, $p_2$..., $p_i$), des indicateurs ($c_1$, $c_2$..., $c_j$) liés à un risque de non-adhérence du patient; et comparer les indicateurs ($c_1$, $c_2$..., cj) à des valeurs-seuils ($s_1$, $s_2$,..., sk) mémorisées de manière à établir un profil spécifique pour le patient considéré correspondant à l'un de plusieurs profils (F1, F2, ..., Fn) mémorisés liés chacun un score prédictif SP de risque de non-adhérence.

Fig. 1

EP 4 374 783 A1

**(Cont. page suivante)**

Fig. 3

| Patient ID | Score de Risque | Criticité |
|---|---|---|
| 345564 | 98% | Elevé |
| 193543 | 95% | Elevé |
| 121236 | 93% | Elevé |
| ... | | |
| 015676 | 71% | Moyen |
| 570337 | 69% | Moyen |
| 307752 | 62% | Moyen |
| ... | | |
| 123977 | 36% | Faible |
| 678932 | 25% | Faible |
| 435589 | 19% | Faible |
| ... | | |

(b) Historique mesures
p1
p2
pi
J0 J1 J2

(c)
ID et Profil du patient 015676
F7
Score prédictif de risque de
non-adhérence : 71%
Causes de non adhérence
pour ce profil :
Fuite masque
Pression inadéquate

(52) Classification Coopérative des Brevets (CPC):
(Cont.)
A61M 2205/3331; A61M 2205/3334;
A61M 2205/3553; A61M 2205/3592;
A61M 2205/505; A61M 2205/52; A61M 2205/581;
A61M 2205/584; A61M 2205/8206;
A61M 2205/8262; G06F 18/24323;
G06F 2218/08; G06F 2218/12

**Description**

L'invention concerne un système permettant de détecter et d'alerter d'un risque de non-adhérence à son traitement, d'un patient souffrant d'apnée obstructive du sommeil.

Le syndrome d'apnée obstructive du sommeil (SAOS) ou plus simplement « l'apnée du sommeil », est un trouble du sommeil qui se caractérise, chez les personnes qui en souffrent, par des pauses respiratoires (apnées) ou des limitations de débit respiratoire (hypopnées) pendant le sommeil, d'une durée de quelques secondes, conduisant à une baisse d'oxygénation du sang mais aussi à des micro-réveils ainsi que d'autres conséquences négatives comme une somnolence pendant la journée, une irritabilité, des réveils en sursaut, une baisse de concentration diurne...

Un traitement classique de l'apnée du sommeil repose sur une administration de gaz sous pression, typiquement d'air, aux voies aériennes de la personne qui en souffre, c'est-à-dire d'un patient, au moyen d'un masque respiratoire, tel un masque nasal, alimenté en gaz sous pression par un conduit flexible véhiculant le gaz sous pression délivré par un appareil d'assistance respiratoire, tel un dispositif de type CPAP (Continuous Positive Airways Pressure) délivrant une pression positive continue de gaz, i.e. de l'air sous pression.

Par exemple, EP-A-2140902 décrit un masque nasal et EP-A-3482788 enseigne un appareil d'assistance respiratoire qui sont utilisables pour traiter l'apnée du sommeil.

L'apnée du sommeil étant un trouble nocturne, il est indispensable de pouvoir accompagner le patient pour qu'il observe bien son traitement pendant la nuit, c'est-à-dire lorsqu'il est à son domicile, en l'absence de tout personnel soignant.

Dans cette optique, EP-A-2542287 propose un dispositif de suivi de l'observance d'un traitement de l'apnée du sommeil installé sur le conduit flexible reliant fluidiquement l'appareil de fourniture de gaz au masque respiratoire délivrant le gaz sous pression au patient. Il permet de mesurer le débit et la pression du gaz et ensuite de traiter ces mesures pour y détecter des évènements respiratoires de type apnée, hypopnée, ronflements ou autres limitations de débit. Toutefois, ce type de dispositif ne s'est pas montré très efficace en pratique et est dès lors peu, voire pas, utilisé.

Par ailleurs, US-A-2017/0209657 enseigne un système permettant d'analyser les données ou mesures provenant d'un appareil de traitement par pression, notamment de l'apnée du sommeil, d'en déduire une prédiction de compliance du patient à son traitement, et ensuite de proposer une ou des actions à mener pour améliorer le traitement du patient, en particulier une action visant à ajuster un programme thérapeutique. Cependant, la détermination des actions n'est pas claire, et l'efficacité du système pas démontrée. De plus, les actions à mener étant déterminées automatiquement et sans interprétation à destination du personnel de santé, c'est-à-dire sans analyse des causes, il peut en résulter des risques pour le patient, ce qui s'oppose à une généralisation de ce type de système.

On connait par ailleurs le document : A Data-Driven Approach for Continuous Adhérence Prédictions in Sleep Apnea Therapy Management ; M. Araujo et al., 2019, IEEE International Conférence on Big Data, p. 2716-2725, qui décrit l'utilisation de plusieurs modèles d'apprentissage machine (machine learning) pour aider les praticiens à prédire un risque de non-adhérence chez des patients traités par des dispositifs de type CPAP.

Le problème est dès lors de proposer un système amélioré permettant de détecter efficacement, de manière proactive, et d'analyser les causes de non-adhérence pour aider le personnel à identifier les actions à mener, et préférentiellement d'alerter ensuite, d'un risque de non-adhérence à son traitement, d'un patient souffrant d'apnée du sommeil, pour lequel un traitement par administration de gaz sous pression, typiquement d'air sous pression, a été prescrit.

La solution de l'invention concerne alors un système pour détecter et alerter d'un risque de non-adhérence à un traitement d'au moins un patient, i.e. une personne humaine, souffrant d'apnée du sommeil comprenant :

- un dispositif de mesure comprenant des moyens de mesure pour opérer des mesures de débit et/ou de pression d'un gaz respiratoire fourni au patient,
- des moyens informatiques de traitement de données configurés pour :

  • traiter les mesures de débit et/ou de pression obtenues sur au moins 3 jours afin de déterminer ou estimer des grandeurs ou valeurs de plusieurs paramètres respiratoires ($p_1$, $p_2$..., $p_i$) du patient considéré choisis parmi au moins une pression, une durée de traitement, un indice d'apnée/hypopnée (IAH) et un débit de fuite,
  • déduire desdits paramètres respiratoires ($p_1$, $p_2$..., $p_i$), des indicateurs ($c_1$, $c_2$..., $c_j$) liés à un risque de non-adhérence du patient et
  • comparer les indicateurs ($c_1$, $c_2$..., $c_j$) à des valeurs-seuils ($s_1$, $s_2$,... $s_k$) mémorisées de manière à établir un profil spécifique pour le patient considéré, ledit profil spécifique correspondant à l'un de plusieurs profils mémorisés liés chacun un score prédictif SP de risque de non-adhérence,

- des moyens de mémorisation pour mémoriser les valeurs-seuils ($s_1$, $s_2$,..., $s_k$) et les profils mémorisés ayant été obtenus à partir d'un historique de plusieurs patients dont l'adhérence au traitement est connue, chaque profil mémorisé étant caractérisé par au moins un score prédictif SP de risque de non-adhérence et

- un dispositif d'affichage pour afficher :

  i) le score prédictif SP de risque de non-adhérence pour ledit au moins un patient considéré, et
  ii) une représentation graphique d'au moins un paramètre respiratoire ($p_1$, $p_2$..., $p_i$) dudit au moins un patient considéré.

De plus, selon l'invention, les moyens informatiques de traitement de données comprennent un serveur informatique et sont en outre configurés pour :

  a) déterminer à partir du profil donné obtenu pour ledit au moins un patient considéré, au moins une cause de non-adhérence choisie parmi des fuites au masque ou un réglage de pression inadéquat, chaque profil mémorisé étant caractérisé par au moins un score prédictif SP de risque de non-adhérence et une cause de non-adhérence, et
  b) commander en outre un affichage de ladite cause de non-adhérence sur le dispositif d'affichage.

Selon le mode de réalisation considéré, l'invention peut comprendre l'une ou plusieurs des caractéristiques suivantes :

- le dispositif de mesure est configuré pour être agencé sur le trajet du gaz entre un appareil de fourniture de gaz sous pression positive et un masque respiratoire, en particulier un masque nasal.
- l'appareil de fourniture de gaz sous pression positive est un appareil de type CPAP.
- le dispositif de mesure est configuré pour être agencé sur le conduit flexible véhiculant le gaz sous pression, tel de l'air pressurisé, depuis l'appareil de fourniture de gaz et le masque respiratoire.
- le dispositif de mesure comprend un boitier contenant un ou plusieurs capteurs de débit ou de pression et préférentiellement une (ou des) source de courant électrique, telle une batterie.
- les moyens informatiques de traitement de données sont configurés pour traiter les mesures de débit et/ou de pression obtenues sur au moins 4 jours, de préférence de 4 à 10 jours, de préférence de l'ordre de 7 jour.
- le dispositif d'affichage est configuré pour afficher simultanément les scores prédictifs SP de risque de non-adhérence de plusieurs patients.
- le dispositif d'affichage est configuré pour afficher les patients regroupés en fonction de leurs scores prédictifs SP de risque, de préférence les groupes de patients sont affichés dans des couleurs différentes.
- le dispositif d'affichage est configuré pour afficher une représentation graphique d'au moins un paramètre respiratoire ($p_1$, $p_2$..., $p_i$) d'un patient considéré sous forme d'une courbe ou d'un barographe sur plusieurs jours.
- il comprend en outre des moyens d'alerte qui coopèrent avec le dispositif d'affichage pour afficher une alerte lorsque le score prédictif SP est supérieur à une valeur de risque donnée, c'est-à-dire un seuil d'alerte prédéterminé, par exemple un score prédictif SP d'au moins 70%, 80% ou 90%.
- les moyens d'alerte sont configurés pour afficher le niveau de risque, par exemple avec un code couleur, et/ou déclencher une alerte sonore.
- les moyens d'alerte peuvent coopérer avec des moyens à haut-parleur pour déclencher une alerte sonore, c'est-à-dire audible.
- les moyens d'alerte sont configurés pour déclencher une alerte chez un prestataire de santé opérant le suivi du patient à distance (PSAD) et éventuellement chez le patient lui-même.
- le dispositif d'affichage est configuré pour afficher une liste de plusieurs patients et d'au moins leur score prédictif SP de risque de non-adhérence, lesdits patients étant classés en fonction de la criticité de leur risque de non-adhérence, c'est-à-dire par priorité, i.e. du plus élevé au moins élevé.
- le dispositif d'affichage est configuré pour afficher la liste de patients classés en fonction de la criticité de leur risque de non-adhérence et en utilisant un code couleur.
- le dispositif d'affichage est configuré pour afficher simultanément pour chaque patient sélectionné, au moins une cause de non-adhérence correspondant à son profil et son score prédictif SP de non-adhérence.
- les moyens informatiques de traitement de données sont en outre configurés pour commander en outre un affichage d'une identification du patient considéré.
- le score prédictif SP de risque de non-adhérence est compris entre 0 et 100%.
- il comprend en outre des moyens de transmission de données pour transmettre à distance les valeurs de paramètres respiratoires ($p_1$, $p_2$..., $p_i$) au serveur informatique (e.g. ordinateur, cloud, edge...), de préférence communiquant à distance avec le dispositif de mesure.
- les moyens informatiques de traitement de données comprennent un ou plusieurs processeurs, notamment un ou des serveurs informatiques comprenant un ou plusieurs processeurs.
- les moyens de transmission de données sont configurés pour transmettre les données ou autres informations, via un protocole de communication, tel que le wifi, le GSM (3G/4G/5G), le Bluetooth®, l'internet, un intranet....

L'invention va maintenant être mieux comprise grâce à la description détaillée suivante, faite à titre illustratif mais non limitatif, en référence aux figures annexées parmi lesquelles :

Fig. 1 schématise un mode de réalisation du système de l'invention,
Fig. 2 schématise un mode de réalisation de la chaîne de décision permettant de prédire le risque de non-adhérence et d'en déterminer les causes, mis en oeuvre par un système selon l'invention, et
Fig. 3 schématise un mode de réalisation des moyens d'affichage et de visualisation des scores de risque, paramètres respiratoires et causes de non-adhérence, mis en oeuvre par un système selon l'invention.

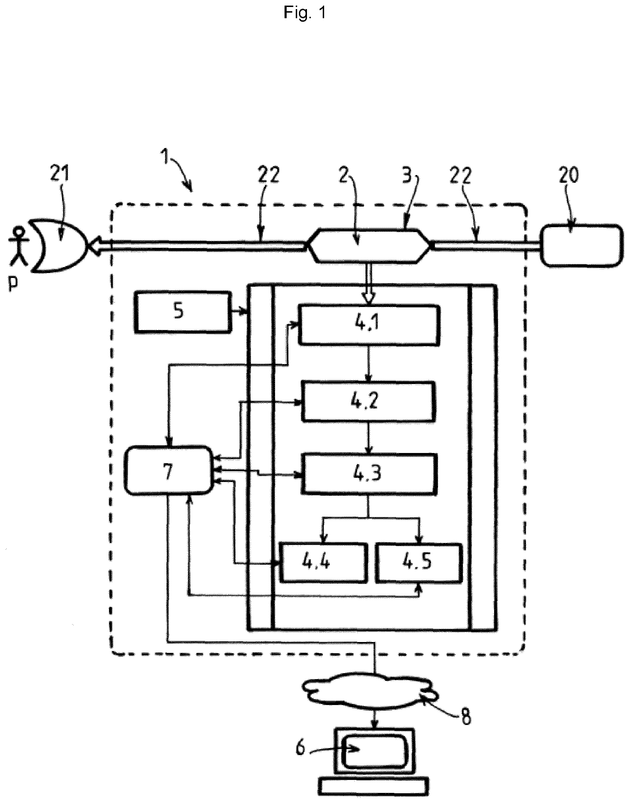

Sur Fig. 1, on a schématisé un patient P souffrant d'apnée du sommeil qui est alimenté en gaz respiratoire sous pression, tel de l'air, fourni par un appareil de fourniture 20 de gaz de type CPAP, pendant les phases de sommeil du patient P, typiquement pendant la nuit.

Le gaz sous pression est acheminé par un conduit flexible 22 depuis l'appareil de fourniture de gaz 20 jusqu'à un masque respiratoire 21 délivrant le gaz aux voies aériennes du patient P, tel un masque nasal.

Le système 1 de l'invention permettant de détecter et d'alerter d'un risque de non-adhérence du patient P à son traitement comprend un dispositif de mesure 2 configuré pour être agencé sur le conduit flexible 22, c'est-à-dire sur le trajet du gaz, afin de permettre d'y opérer des mesures, par exemple de pression et/ou de débit, et déterminer ensuite des valeurs de plusieurs paramètres respiratoires ($p_1$, $p_2$..., $p_i$) relatifs au patient P, tel qu'une ou des pression, durée de traitement, indice d'apnée/hypopnée (IAH), débit de fuites d'air au niveau du masque... Calculer de tels paramètres respiratoires est connu en soi, notamment de EP-A-2542287.

Pour ce faire, le dispositif de mesure 2 comprend un boîtier 3 incluant des moyens de mesure adaptés, tels un ou des capteurs de débit, de pression ou autre, ainsi qu'une source de courant interne, telle qu'une pile ou batterie, alimentant notamment les moyens de mesure.

On prévoit par ailleurs des moyens informatiques de traitement de données 4, tel un processeur, permettant de traiter les valeurs des paramètres respiratoires ($p_1$, $p_2$... $p_i$) mesurées par le dispositif de mesure 2 et en déduire des caractéristiques représentatives ($c_1$,$c_2$...,$c_j$) d'un risque de non-adhérence du patient P, lesquelles sont ensuite comparées à des valeurs-seuils ($s_1$, $s_2$,...$s_k$) mémorisées au sein de moyens de mémorisation, comme une mémoire (disque dur, mémoire flash, stockage cloud ... ) ou autre, afin d'en déduire un score prédictif SP de risque de non-adhérence pour le patient P compris entre 0 et 100%.

Les moyens informatiques de traitement de données 4 sont alimentées électriquement par des moyens de fourniture de courant électrique 5, tel le réseau électrique (110/220V) ou une (ou des) pile(s) ou batterie(s) de stockage.

Par ailleurs, le système 1 de l'invention comprend aussi des moyens d'affichage, aussi appelés dispositif d'affichage 6, permettant d'afficher des informations, des données... par exemple un écran d'affichage d'un ordinateur fixe ou portable, d'un téléphone multifonction (smartphone), d'une tablette numérique ou autre.

Le dispositif d'affichage 6 reçoit les données à afficher via un système ou des moyens de communication 8 adaptés, par exemple des moyens de transmission de données configurés pour transmettre les données ou autres informations, via un protocole de communication, tel que le wifi, le GSM (3G/4G/5G), le Bluetooth®, l'internet, un intranet ou autre.

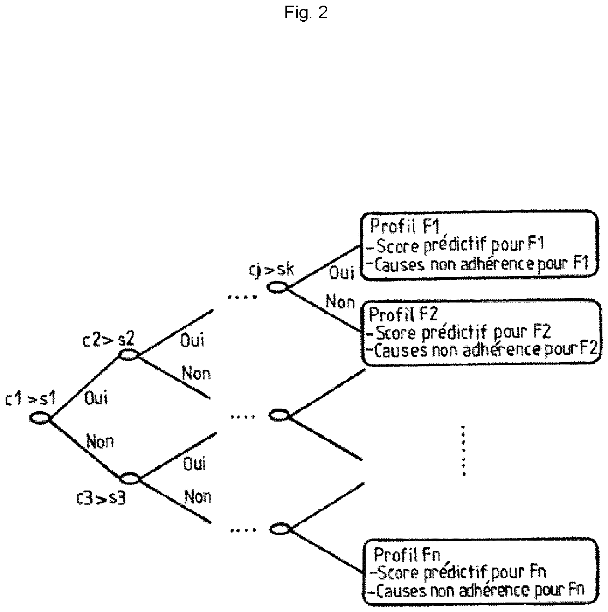

Fig. 2 schématise la chaîne décisionnelle permettant de prédire le risque de non-adhérence du patient P et d'en identifier les causes, laquelle est mise en oeuvre par le système selon l'invention, tel celui de Fig. 1.

Comme on le voit, une fois que le dispositif de mesure 2 du système de l'invention 1 a opéré des mesures de débit et/ou de pression du gaz respiratoire fourni au patient, tel de l'air, pendant une durée d'au moins 3 jours, préférentiellement entre 4 et 8 jours, les moyens informatiques de traitement de données 4, tel un serveur distant, traitent ces mesures de débit et/ou de pression obtenues sur au moins 3 jours pour déterminer ou estimer des grandeurs ou valeurs de plusieurs paramètres respiratoires $p_1$, $p_2$..., $p_i$ du patient P.

Les paramètres respiratoires $p_1$, $p_2$..., $p_i$ ainsi déterminés sont (au moins) une pression, une durée de traitement, un indice d'apnée/hypopnée (IAH) et un débit de fuite.

Ces grandeurs ou valeurs de plusieurs paramètres respiratoires $p_1$, $p_2$..., $p_i$ sont utilisés ensuite par les moyens informatiques de traitement de données 4, pour en déduire des indicateurs $c_1$,$c_2$...,$c_j$ liés à un risque de non-adhérence du patient et les comparer à des valeurs-seuils $s_1$, $s_2$,...,$s_k$ mémorisées de manière à établir un profil spécifique pour le patient P considéré.

Ce profil spécifique correspond en fait à l'un de plusieurs profils F1, F2, ..., Fn mémorisés, qui sont liés chacun à un score prédictif SP de risque de non-adhérence donné.

Ces différents profils F1, F2, ..., Fn prédéterminés, scores prédictifs SP liés et les valeurs-seuils $s_1$, $s_2$,..., $s_k$ sont mémorisés par des moyens de mémorisation, de préférence par le serveur lui-même. Ils ont été obtenus à partir d'un historique de plusieurs patients dont l'adhérence au traitement est connue.

Chaque profil mémorisé F1, F2, ..., Fn est caractérisé par (au moins) un score prédictif SP de risque de non-adhérence.

Autrement dit, en procédant ainsi, grâce aux différents profils F1, F2, ..., Fn prédéterminés, c'est-à-dire connus et enregistrés, il est possible de déterminer aisément à quel profil exact correspond le profil spécifique du patient P considéré et donc quel est le score prédictif SP de risque de non-adhérence de ce patient P qui est typiquement compris entre 0 et 100%.

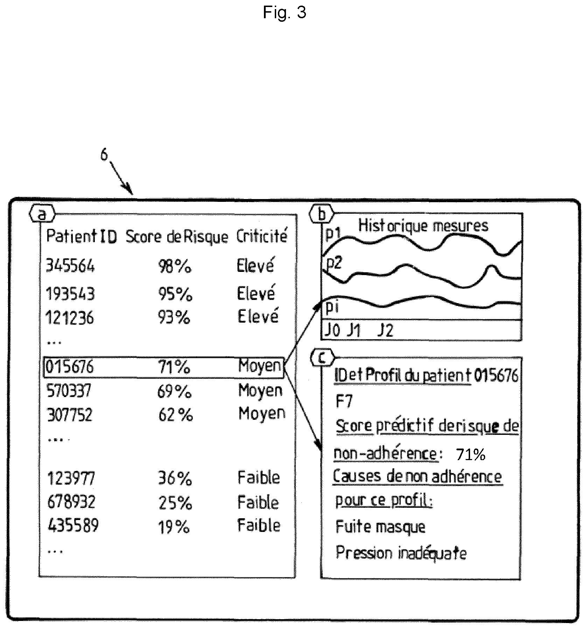

Ensuite, comme illustré en Fig. 3, le dispositif d'affichage 6 du système 1 de l'invention est configuré pour afficher le score prédictif SP de risque de non-adhérence pour le ou les patients considérés, par exemple ici pour au moins 9 patients identifiés chacun par une identification propre (PatientID).

Sont affichés aussi le score de risque SP (en %) de non-adhérence et la criticité (élevée, moyenne, faible) de ce score de risque SP pour chaque patient considéré.

De plus, le dispositif d'affichage 6 du système 1 de l'invention est aussi configuré pour afficher une représentation graphique, tel une ou des courbes en fonction du temps, d'un (ou plusieurs) paramètre respiratoire ($p_1$, $p_2$..., $p_i$) d'un patient sélectionné parmi l'ensemble des patients affichés.

Pour ce faire, un utilisateur, typiquement un personnel soignant, sélectionne le patient particulier pour lequel il souhaite avoir plus d'informations, en particulier pouvoir visualiser l'évolution de ce (ou ces) paramètre respiratoire ($p_1$, $p_2$..., $p_i$). Lorsque cette sélection est opérée, le dispositif d'affichage 6 opère l'affichage désirée de la représentation graphique, par exemple ici des courbes de plusieurs paramètres du patient faisant office d'historique de mesures sur plusieurs jours, typiquement entre 4 et 8 jours.

La représentation graphique est préférentiellement associée à d'autres informations du patient, notamment son identification (PatientID), son profil (F), son score prédictif SP de non-adhérence (%), et avantageusement une ou des causes de non-adhérence au traitement déterminées pour le patient considéré.

Les causes de non-adhérence sont préférentiellement des fuites au masque ou un réglage de pression inadéquat, mais aussi d'autres paramètres.

Les causes de non-adhérence sont déterminées par les moyens informatiques de traitement de données 4 à partir du profil F obtenu pour le patient considéré.

En fait, chaque profil mémorisé est caractérisé par (au moins) un score prédictif SP de risque de non-adhérence et une cause de non-adhérence.

Avantageusement, pour faciliter la lecture et permettre à l'utilisateur, c'est-à-dire le personnel soignant, de distinguer rapidement les patients les plus à risque de ne pas adhérer à leur traitement, le dispositif d'affichage 6 du système 1 de l'invention est aussi configuré pour afficher les patients en une liste de plusieurs patients classés or organisés en fonction de la criticité de leur risque de non-adhérence, c'est-à-dire par priorité, i.e. du plus élevé (en tête de liste) au moins élevé (en bas de liste), comme illustré en Fig. 3.

Cette liste de patients peut aussi être affichée en utilisant un code couleur, par exemple les patients présentant une criticité élevée peuvent être affichés en rouge, ceux présentant une criticité moyenne peuvent être affichés en orange et ceux présentant une criticité basse peuvent être affichés en vert.

La description suivante permet de mieux comprendre l'invention.

Comme illustré en Fig. 1, les moyens informatiques de traitement de données 4 récupèrent et pré-traitent (en 4.1) d'abord les paramètres respiratoires ($p_1$, $p_2$..., $p_i$) relatifs au patient P, tel qu'une ou des pression, durée de traitement, indice d'apnée/hypopnée (IAH) ou débit de fuites. Par exemple, le prétraitement (4.1) peut comprendre des étapes de :

a) Mise à l'échelle des valeurs des paramètres respiratoires (p1, p2..., pi) en fonction de la machine CPAP utilisée et de ses caractéristiques intrinsèques, par exemple pression médiane ou moyenne, grandeurs exprimées en % ou en valeur absolue.... Ainsi, pour un paramètre respiratoire p_i et d'une machine donnée m, on obtient: $q\_i(m) = p\_i(m) - moyenne(p\_i, m) / std(p\_i, m)$

où :

- moyenne(p_i, m) est la moyenne empirique du paramètre respiratoire pi pour la machine m considérée (i.e. selon le constructeur).
- std(p_i, m) est l'écart-type du paramètre respiratoire p_i pour la machine m considérée.
- q_i est la valeur du paramètre respiratoire mise à l'échelle.

b) Remplacement des valeurs manquantes dans les paramètres respiratoires ($p_1$, $p_2$.., $p_i$). Pour un paramètre respiratoire p_i dont la valeur est manquante à un point t, plusieurs méthodes peuvent être appliquées:

- Imputation à valeur fixe: p_i(t) = 0
- Imputation par la moyenne: p_i(t) = moyenne(pi)
- Imputation par la valeur précédente: p_i(t) = p_i(t-1).

c) Encodage des informations liées au patient (genre) en les regroupant par catégories et en calculant une moyenne.

Pour l'information sur le genre, l'encodage se fait par la moyenne du paramètre respiratoire p_i pour chaque modalité de la catégorie (femme, homme) :

Encodage(genre=femme) = moyenne(p_i, genre=femme)
Encodage(genre=homme) = moyenne(p_i, genre=homme)
Ensuite, ces paramètres respiratoires ($p_1$, $p_2$..., $p_i$) sont traités et on en déduit les caractéristiques représentatives ($c_1$, $c_2$...,$c_j$), aussi appelées indicateurs statistiques, qui sont enfin comparées (en 4.2) aux valeurs-seuils ($s_1$, $s_2$,...$s_k$) mémorisées (en 7) pour déterminer (en 4.3) le score prédictif SP de risque de non-adhérence.

Par exemple, le traitement (4.2) comprend des étapes de calcul d'indicateurs statistiques sur les paramètres respiratoires $p_i$(IAH, observance, fuites, pression), par exemple détermination de moyenne, tendance ... comme donné dans Tab. 1 suivant.

Tab. 1

| | Caractéristiques représentatives ($c_1$, $c_2$...,$c_j$) | Description |
|---|---|---|
| Informations patient | Genre | Homme, Femme |
| | Age | Age du patient |
| | Durée | Durée du traitement |
| Paramètres respiratoires pi | Fuites | Moyenne |
| | | Variance |
| | | Tendance |
| | | Excentricité (asymétrie) |
| | Observance | Moyenne |
| | | Variance |
| | | Tendance |
| | | Excentricité (asymétrie) |
| | IAH | Moyenne |
| | | Variance |
| | | Tendance |
| | | Excentricité (asymétrie) |
| | Pression | Moyenne |
| | | Variance |
| | | Tendance |
| | | Excentricité (asymétrie) |

Ces indicateurs statistiques sont calculés par exemple via les formules suivantes où D est le nombre total de jours t, et X(t) est le paramètre respiratoire relevé à un jour t donné. Ainsi :

La moyenne $\mu$ est calculée à partir de l'équation : $\mu = (1/D) . \sum_t X(t)$
L'écart-type $\sigma$ est calculé à partir de l'équation : $\sigma = (1/D) . \sum_t (X - \mu)^2$
La variance V correspond à : $V = \sigma^2$
La tendance à correspond à : $\hat{a}, \hat{B} = \text{argmin} \sum_t (X(t) - (at + b))^2$
L'excentricité S (l'asymétrie) correspond à : $S = \mu^3 / \sigma^3$

Le score prédictif SP obtenu sert à déclencher une alerte de non-adhérence (en 4.4) et par ailleurs à calculer un profil de risque (en 4.5) pour le patient considéré.

L'alerte de non-adhérence (4.4) et le profil de risque (4.5) peuvent être transmis au dispositif d'affichage 6, tel un écran d'ordinateur ou de téléphone multifonction (smartphone), qui permet de l'afficher. La transmission du score SP peut se faire via un protocole et/ou réseau de communication 8, tel que web, GSM ou autre.

Le déclenchement d'une alerte de non-adhérence (en 4.4) se fait préférentiellement via un processus de Machine Learning (apprentissage machine).

Ce processus utilise un historique de données comprenant au moins les paramètres respiratoires préalablement collectés sur des patients dont on connaît l'issue, c'est-à-dire pour lequel on sait s'ils ont été adhérents ou non-adhérentes au traitement, après la période cible de 2 semaines et 3 mois, typiquement 1 mois.

Une méthode mathématique utilisable, comme illustré en Fig. 2, repose sur une priorisation des caractéristiques représentatives ($c_1$, $c_2$...,Cj) extraites des paramètres respiratoires, de sorte que les premières soient celles ayant un pouvoir discriminant plus important. Plus précisément, une caractéristique $c$ est choisie comme prioritaire si elle permet de séparer à l'aide d'un seuil $s$, le plus grand nombre de patients en deux groupes, à savoir un groupe de patients majoritairement adhérents et un groupe de patients majoritairement non-adhérents.

La détermination de la caractéristique prioritaire $c$ et le choix optimal du seuil s sont effectués avec un algorithme d'optimisation, comme celui décrit par : Leo Breiman, Classification and régression trees, Monterey, CA, Wadsworth & Brooks/Cole Advanced Books & Software, 1984, 368 p. (ISBN 978-0-412-04841-8*)*

Cette méthode est appliquée séquentiellement à chaque groupe ainsi obtenu pour déterminer la caractéristique prioritaire et son seuil optimal de ces groupes, et obtenir alors 2 nouveaux sous-groupes. Ceci est répété plusieurs fois de suite.

Le processus s'arrête avec un critère d'arrêt basé sur le nombre d'itérations ou lorsque les sous-groupes de patients correspondent à des profils suffisamment homogènes de patients, c'est-à-dire des patients qui sont principalement adhérents ou principalement non adhérents. Ces profils suffisamment homogènes comprennent tous les profils Fn, i.e. F1, F2, ..., Fn.

Le score prédictif SP_n associé à chaque profil Fn est calculé comme étant la proportion des patients non-adhérents suivant la formule:

$$SP\_n(Profil\ Fn) = Nb\text{-}P\ non\text{-}adhérents\ (Profil\ Fn)\ /\ Nb\text{-}P\ (Profil\ Fn)$$

où : Nb-P est le nombre de patients

Enfin, les patients de chaque profil Fn de la base de données étant préalablement bien connus par le personnel soignant, les causes principales de leur non-adhérence sont généralement connues car communes à tous les patients d'un même profil (Fn).

De plus, pour déterminer pour chaque prédiction les facteurs les plus importants, c'est-à-dire ceux ayant conduit à la non-adhérence du patient, on peut utiliser un ou des algorithmes d'apprentissage machine ou Machine Learning, par exemple ceux décrits par :

- M. T. Ribeiro, S. Singh, C. Guestrin, Anchors: high-precision model-agnostic explanations, AAAI conférence on artificial intelligence (AAAI); 2018*, ou*
- M. T. Ribeiro, S. Singh, C. Guestrin, why should 1 trust you?" explaining the prédictions of any classifier, Proceedings of the 22nd ACM SIGKDD international conférence on knowledge discovery and data mining; 2016, pp. 1135-1144*.*

A partir des scores prédictifs, trois niveaux de criticités du risque de non-adhérence sont affichés sur Fig. 3, lesquels peuvent être définis avec ou par le personnel soignant.

L'alerte de non-adhérence peut être visuelle et/ou sonore. A cette fin, le système de l'invention peut comprendre des moyens d'affichage visuel et/ou des moyens sonores.

D'une façon plus générale, le système de l'invention permet de détecter et d'alerter d'un risque de non-adhérence à un traitement d'un patient P souffrant d'apnée obstructive du sommeil se caractérisant par des pauses respiratoires (apnées) ou des limitations de débit respiratoire (hypopnées) pendant le sommeil du patient P, d'une durée de quelques secondes, ledit patient P étant traité par administration de gaz sous pression, tel de l'air, délivré par un appareil d'assistance respiratoire de type CPAP.

**Revendications**

1. Système (1) pour détecter et alerter d'un risque de non-adhérence à un traitement d'au moins un patient (P) souffrant d'apnée du sommeil comprenant :

- un dispositif de mesure (2) comprenant des moyens de mesure pour opérer des mesures de débit et/ou de pression d'un gaz respiratoire fourni au patient,
- des moyens informatiques de traitement de données (4) configurés pour :

. traiter les mesures de débit et/ou de pression obtenues sur au moins 3 jours afin de déterminer ou estimer des grandeurs ou valeurs de plusieurs paramètres respiratoires ($p_1$, $p_2$..., $p_i$) du patient considéré choisis parmi au moins une pression, une durée de traitement, un indice d'apnée/hypopnée (IAH) et un débit de fuite,
. déduire desdits paramètres respiratoires ($p_1$, $p_2$..., $p_i$), des indicateurs ($c_1$,$c_2$...,$c_j$) liés à un risque de non-adhérence du patient, et
. comparer les indicateurs ($c_1$, $c_2$...,$c_j$) à des valeurs-seuils ($s_1$, $s_2$,...,sk) mémorisées de manière à établir un profil spécifique pour le patient considéré, ledit profil spécifique correspondant à l'un de plusieurs profils (F1, F2, ..., Fn) mémorisés liés chacun un score prédictif SP de risque de non-adhérence,

- des moyens mémorisation pour mémoriser les valeurs-seuils ($s_1$, $s_2$,..., sk) et les profils mémorisés ayant été obtenus à partir d'un historique de plusieurs patients dont l'adhérence au traitement est connue, chaque profil mémorisé étant **caractérisé par** au moins un score prédictif SP de risque de non-adhérence et
- un dispositif d'affichage (6) pour afficher :

i) le score prédictif SP de risque de non-adhérence pour ledit au moins un patient considéré, et
ii) une représentation graphique d'au moins un paramètre respiratoire ($p_1$, $p_2$..., $p_i$) dudit au moins un patient considéré,
**caractérisé en ce que** les moyens informatiques de traitement de données (4) comprennent un serveur informatique et sont configurés pour :

a) déterminer à partir du profil donné obtenu pour ledit au moins un patient considéré, au moins une cause de non-adhérence choisie parmi des fuites au masque ou un réglage de pression inadéquat, chaque profil mémorisé étant **caractérisé par** au moins un score prédictif SP de risque de non-adhérence et une cause de non-adhérence, et
b) commander en outre un affichage de ladite cause de non-adhérence sur le dispositif d'affichage (6).

**2.** Système selon la revendication 1, caractérisé ce que le dispositif de mesure (2) est agencé sur le trajet du gaz entre un appareil de fourniture de gaz sous pression positive et un masque respiratoire.

**3.** Système selon la revendication 1, **caractérisé en ce qu'**il comprend en outre des moyens d'alerte qui coopèrent avec le dispositif d'affichage (6) pour afficher une alerte lorsque le score prédictif SP est supérieur à une valeur de risque donnée.

**4.** Système selon la revendication 2, **caractérisé en ce que** l'appareil de fourniture de gaz sous pression positive est un appareil de type CPAP.

**5.** Système selon la revendication 1, **caractérisé en ce que** le dispositif d'affichage (6) est configuré pour afficher simultanément les scores prédictifs SP de risque de non-adhérence de plusieurs patients.

**6.** Système selon la revendication 5, **caractérisé en ce que** le dispositif d'affichage (6) est configuré pour afficher les patients regroupés en fonction de leurs scores prédictifs SP de risque, de préférence les groupes de patients sont affichés dans des couleurs différentes.

**7.** Système selon la revendication 1, **caractérisé en ce que** le dispositif d'affichage (6) est configuré pour afficher une représentation graphique d'au moins un paramètre respiratoire ($p_1$, $p_2$..., $p_i$) d'un patient considéré sous forme d'une courbe ou d'un barographe sur plusieurs jours.

**8.** Système selon la revendication 1, **caractérisé en ce qu'**il comprend en outre des moyens de transmission de données (8) configurés pour transmettre à distance les valeurs de paramètres respiratoires ($p_1$, $p_2$..., $p_i$) au serveur informatique communiquant à distance avec le dispositif de mesure.

**9.** Système selon la revendication 1, **caractérisé en ce que** le dispositif d'affichage (6) est configuré pour afficher simultanément pour chaque patient sélectionné, au moins une cause de non-adhérence correspondant à son profil et son score prédictif SP de non-adhérence.

**10.** Système selon l'une des revendications 1 ou 2, **caractérisé en ce que** le dispositif de mesure (2) comprend un boitier contenant un ou plusieurs capteurs de débit ou de pression.

**11.** Système selon l'une des revendications 1 ou 9, **caractérisé en ce que** les moyens informatiques de traitement de données (4) sont en outre configurés pour commander en outre un affichage d'une identification du patient considéré.

**12.** Système selon la revendication 6, **caractérisé en ce que** le dispositif d'affichage (6) est configuré pour afficher une liste de plusieurs patients classés du risque le plus élevé au risque le moins élevé.

**13.** Système selon la revendication 1, **caractérisé en ce que** les moyens informatiques de traitement de données () sont configurés pour traiter les mesures de débit et/ou de pression obtenues sur de 4 à 10 jours.

**14.** Système selon la revendication 1, **caractérisé en ce qu'**il comprend en outre des moyens d'alerte qui coopèrent avec le dispositif d'affichage (6) pour afficher une alerte lorsque le score prédictif SP est supérieur à une valeur de risque donnée.

**15.** Système selon la revendication 14, **caractérisé en ce que** les moyens d'alerte sont configurés pour déclencher une alerte chez un prestataire de santé opérant le suivi du patient à distance (PSAD).

**Revendications modifiées conformément à la règle 137(2) CBE.**

**1.** Système (1) pour détecter et alerter d'un risque de non-adhérence à un traitement d'au moins un patient (P) souffrant d'apnée du sommeil comprenant :

- un dispositif de mesure (2) comprenant des moyens de mesure pour opérer des mesures de débit et/ou de pression d'un gaz respiratoire fourni au patient,
- des moyens informatiques de traitement de données (4) configurés pour :

. traiter les mesures de débit et/ou de pression obtenues sur au moins 3 jours afin de déterminer ou estimer des grandeurs ou valeurs de plusieurs paramètres respiratoires ($p_1$, $p_2$..., $p_i$) du patient considéré choisis parmi au moins une pression, une durée de traitement, un indice d'apnée/hypopnée (IAH) et un débit de fuite,
. déduire desdits paramètres respiratoires ($p_1$, $p_2$..., $p_i$), des indicateurs ($c_1$,$c_2$...,$c_j$) liés à un risque de non-adhérence du patient, et
. comparer les indicateurs ($c_1$, $c_2$...,$c_j$) à des valeurs-seuils ($s_1$, $s_2$,...,$s_k$) mémorisées de manière à établir un profil spécifique pour le patient considéré, ledit profil spécifique correspondant à l'un de plusieurs profils (F1, F2, ..., Fn) mémorisés liés chacun un score prédictif SP de risque de non-adhérence,

- des moyens mémorisation pour mémoriser les valeurs-seuils ($s_1$, $s_2$,..., $s_k$) et les profils mémorisés ayant été obtenus à partir d'un historique de plusieurs patients dont l'adhérence au traitement est connue, chaque profil mémorisé étant **caractérisé par** au moins un score prédictif SP de risque de non-adhérence et
- un dispositif d'affichage (6) pour afficher :

i) le score prédictif SP de risque de non-adhérence pour ledit au moins un patient considéré, et
ii) une représentation graphique d'au moins un paramètre respiratoire ($p_1$, $p_2$..., $p_i$) dudit au moins un patient considéré,
dans lequel les moyens informatiques de traitement de données (4) sont configurés pour :

a) déterminer à partir du profil donné obtenu pour ledit au moins un patient considéré, au moins une cause de non-adhérence choisie parmi des fuites au masque ou un réglage de pression inadéquat, chaque profil mémorisé étant **caractérisé par** au moins un score prédictif SP de risque de non-adhérence et une cause de non-adhérence, et
b) commander en outre un affichage de ladite cause de non-adhérence sur le dispositif d'affichage (6), caractérisé ce que :

- les moyens informatiques de traitement de données (4) comprennent un serveur informatique et
- le système comprend en outre des moyens d'alerte qui coopèrent avec le dispositif d'affichage (6) pour afficher une alerte lorsque le score prédictif SP est supérieur à une valeur de risque donnée, et déclencher une alerte chez un prestataire de santé opérant le suivi du patient à distance (PSAD).

2. Système selon la revendication 1, caractérisé ce que le dispositif de mesure (2) est agencé sur le trajet du gaz entre un appareil de fourniture de gaz sous pression positive et un masque respiratoire.

3. Système selon la revendication 1, **caractérisé en ce qu'**il comprend en outre des moyens d'alerte qui coopèrent avec le dispositif d'affichage (6) pour afficher une alerte lorsque le score prédictif SP est supérieur à une valeur de risque donnée.

4. Système selon la revendication 2, **caractérisé en ce que** l'appareil de fourniture de gaz sous pression positive est un appareil de type CPAP.

5. Système selon la revendication 1, **caractérisé en ce que** le dispositif d'affichage (6) est configuré pour afficher simultanément les scores prédictifs SP de risque de non-adhérence de plusieurs patients.

6. Système selon la revendication 5, **caractérisé en ce que** le dispositif d'affichage (6) est configuré pour afficher les patients regroupés en fonction de leurs scores prédictifs SP de risque, de préférence les groupes de patients sont affichés dans des couleurs différentes.

7. Système selon la revendication 1, **caractérisé en ce que** le dispositif d'affichage (6) est configuré pour afficher une représentation graphique d'au moins un paramètre respiratoire ($p_1$, $p_2$..., $p_i$) d'un patient considéré sous forme d'une courbe ou d'un barographe sur plusieurs jours.

8. Système selon la revendication 1, **caractérisé en ce qu'**il comprend en outre des moyens de transmission de données (8) configurés pour transmettre à distance les valeurs de paramètres respiratoires ($p_1$, $p_2$..., $p_i$) au serveur informatique communiquant à distance avec le dispositif de mesure.

9. Système selon la revendication 1, **caractérisé en ce que** le dispositif d'affichage (6) est configuré pour afficher simultanément pour chaque patient sélectionné, au moins une cause de non-adhérence correspondant à son profil et son score prédictif SP de non-adhérence.

10. Système selon l'une des revendications 1 ou 2, **caractérisé en ce que** le dispositif de mesure (2) comprend un boitier contenant un ou plusieurs capteurs de débit ou de pression.

11. Système selon l'une des revendications 1 ou 9, **caractérisé en ce que** les moyens informatiques de traitement de données (4) sont en outre configurés pour commander en outre un affichage d'une identification du patient considéré.

12. Système selon la revendication 6, **caractérisé en ce que** le dispositif d'affichage (6) est configuré pour afficher une liste de plusieurs patients classés du risque le plus élevé au risque le moins élevé.

13. Système selon la revendication 1, **caractérisé en ce que** les moyens informatiques de traitement de données (4) sont configurés pour traiter les mesures de débit et/ou de pression obtenues sur de 4 à 10 jours.

Fig. 1

Fig. 2

**Profil F1**
-Score prédictif pour F1
-Causes non adhérence pour F1

**Profil F2**
-Score prédictif pour F2
-Causes non adhérence pour F2

**Profil Fn**
-Score prédictif pour Fn
-Causes non adhérence pour Fn

$c_1 > s_1$

$c_2 > s_2$

$c_3 > s_3$

$c_j > s_k$

Oui

Non

Fig. 3

# EP 4 374 783 A1

Europäisches
Patentamt

European
Patent Office

Office européen
des brevets

## RAPPORT DE RECHERCHE EUROPEENNE

Numéro de la demande

EP 23 20 7096

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | ARAUJO MATHEUS ET AL: "A Data-Driven Approach for Continuous Adherence Predictions in Sleep Apnea Therapy Management", 2019 IEEE INTERNATIONAL CONFERENCE ON BIG DATA (BIG DATA), IEEE, 9 décembre 2019 (2019-12-09), pages 2716-2725, XP033721642, DOI: 10.1109/BIGDATA47090.2019.9006476 * figures 1,2,4,8 * * abrégé * * section III * * section IV * * le document en entier * ----- | 1-15 | INV. A61B5/08 A61M16/00 |
| A | US 2014/032231 A1 (SEMEN TIMOTHY [AU] ET AL) 30 janvier 2014 (2014-01-30) * figures 1-3 * * le document en entier * ----- | 4,7,8 | |
| A | US 2013/331726 A1 (WEBER CLAUDE [FR]) 12 décembre 2013 (2013-12-12) * alinéas [0049], [0053] − [0054], [0063], [0066] − [0071], [0095]; figures 1-2 * ----- | 1-4,10, 13 | DOMAINES TECHNIQUES RECHERCHES (IPC) A61B G06V A61M G06F G16H |

Le présent rapport a été établi pour toutes les revendications

| Lieu de la recherche | Date d'achèvement de la recherche | Examinateur |
|---|---|---|
| La Haye | 25 janvier 2024 | Sarcia, Regis |

**ANNEXE AU RAPPORT DE RECHERCHE EUROPEENNE**
**RELATIF A LA DEMANDE DE BREVET EUROPEEN NO.**

**EP 23 20 7096**

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

**25-01-2024**

| Document brevet cité au rapport de recherche | Date de publication | Membre(s) de la famille de brevet(s) | | Date de publication |
|---|---|---|---|---|
| US 2014032231 A1 | 30-01-2014 | AU | 2013200972 A1 | 13-02-2014 |
| | | US | 2014032231 A1 | 30-01-2014 |
| | | US | 2020143939 A1 | 07-05-2020 |
| | | US | 2023046245 A1 | 16-02-2023 |
| US 2013331726 A1 | 12-12-2013 | AU | 2012220479 A1 | 25-07-2013 |
| | | CA | 2823861 A1 | 30-08-2012 |
| | | CN | 103391793 A | 13-11-2013 |
| | | EP | 2678061 A1 | 01-01-2014 |
| | | FR | 2971930 A1 | 31-08-2012 |
| | | JP | 2014508595 A | 10-04-2014 |
| | | US | 2013331726 A1 | 12-12-2013 |
| | | WO | 2012114004 A1 | 30-08-2012 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2140902 A
- EP 3482788 A
- EP 2542287 A
- US 20170209657 A

**Littérature non-brevet citée dans la description**

- **M. ARAUJO et al.** A Data-Driven Approach for Continuons Adhérence Prédictions in Sleep Apnea Therapy Management. *IEEE International Conférence on Big Data,* 2019, 2716-2725
- Classification and régression trees. **LEO BREIMAN.** Cole Advanced Books & Software. Wadsworth & Brooks, 1984, 368
- **M. T. RIBEIRO ; S. SINGH ; C. GUESTRIN.** Anchors: high-precision model-agnostic explanations. *AAAI conférence on artificial intelligence (AAAI),* 2018
- **M. T. RIBEIRO ; S. SINGH ; C. GUESTRIN.** *Proceedings of the 22nd ACM SIGKDD international conférence on knowledge discovery and data mining;,* 2016, 1135-1144